(19) Europäisches Patentamt
European Patent Office
Office européen des brevets

(11) Veröffentlichungsnummer: **0 003 122**
**B1**

(12) # EUROPÄISCHE PATENTSCHRIFT

(45) Veröffentlichungstag der Patentschrift: **29.04.81**

(51) Int. Cl.³: **C 07 C 103/52**

(21) Anmeldenummer: **79100048.2**

(22) Anmeldetag: **09.01.79**

(54) **Verfahren zur Herstellung cysteinhaltiger Peptide.**

(30) Priorität: **12.01.78 DE 2801175**

(43) Veröffentlichungstag der Anmeldung: **25.07.79 Patentblatt 79/15**

(45) Bekanntmachung des Hinweises auf die Patenterteilung: **29.04.81 Patentblatt 81/17**

(84) Benannte Vertragsstaaten: **BE CH DE FR GB**

(56) Entgegenhaltungen:
**DE-A-1 964 798**
**GB-A-1 243 035**
**METHODEN DER ORGANISCHEN CHEMIE, IV. Auflage, Houben—Weyl, Band XV/1, 1. Teil »Synthese von Peptiden«, 1974, Georg Thieme Verlag, Stuttgart, DE**
*** Seiten 749—758 ***

(73) Patentinhaber: **HOECHST AKTIENGESELLSCHAFT, Zentrale Patentabteilung Postfach 80 03 20, D-6230 Frankfurt/Main 80 (DE)**

(72) Erfinder: **König, Wolfgang, Dr., Eppsteiner Strasse 25, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen)

BUNDESDRUCKEREI BERLIN

EP 0 003 122 B1

## Verfahren zur Herstellung cysteinhaltiger Peptide

Bei der Synthese cysteinhaltiger Peptide hat der Tritylrest zum Schutz der Mercaptofunktion an steigender Bedeutung gewonnen. Die sauer Abspaltung dieser Schutzgruppe stieß bei höhermulekularen Peptiden jedoch auf Schwierigkeiten. So konnten von einer Insulin-A-Kette, deren 4-Cysteingruppen durch Tritylgruppen geschützt waren, nur unter wiederholten Behandlungen mit HBr und Trifluoressigsäure oder HF die Schutzgruppen abgespalten werden (vgl. Z. Naturforsch., 24b, 1128 [1969]). Die Ausbeute an Insulin-A-Ketten-tetrasulfonate bei vollgeschützter Kette betrug nur 21%.

Reine Trifluoressigsäure bewirkt keinerlei Abspaltung (Chem. Ber., 101, 681 [1968]); nachdem die Trifluoressigsäurelösung der S-Tritylverbindung jedoch in Wasser gegossen wurde, konnte bei H-Cys(Trt)-OH eine 70–75%ige Abspaltung beobachtet werden. Bei Peptiden war die Abspaltrate aber nur maximal 60%ig (J. Chem. Soc. [C], 1970, 2683).

Die S-Tritylgruppe bei cysteinhaltigen Peptiden wird daher meist durch Jodoxydation in organischen Lösungsmitteln (J. Chem. Soc., 74 [1952], 1862; Helv. Chim. Acty, 51 [1968], 2061) oder in wäßriger Essigsäure (DE-B-1 917 939) abgespalten. Bei dieser Oxydation entsteht direkt das entsprechende Cystinderivat, eine Deblockierung unter Regenerierung der Mercaptofunktion ist somit auf diesem Weg nicht möglich. Ein weiterer Nachteil dieser Methode ist, daß Jod als starkes Oxydationsmittel bei oxydationsempfindichen Peptiden Nebenprodukte bilden kann.

Während sich Trifluoressigsäuremischungen mit Wasser oder dem Kationenfänger Anisol die Abspaltung der S-Tritylgruppe nachteilig beeinflussen, wurde überraschenderweise gefunden, daß Mischungen aus Trifluoressigsäure mit Mercaptanen ein ausgezeichnetes und mildes Abspaltungsmedium für S-Tritylgruppen darstellen.

Gegenstand der Erfindung ist ein Verfahren zur Herstellung von cysteinhaltigen Peptiden durch Abspaltung der S-Tritylgruppen mittels Trifluoressigsäure, dadurch gekennzeichnet, daß man S-Tritylverbindungen dieser Peptide mit einer Mischung aus einem Mercaptan und Trifluoressigsäure bei einer Trifluoressigsäurekonzentration von mindestens 30 Volumprozent behandelt.

Als Mercaptane kommen sowohl aliphatische als auch aromatische Mercaptane, wie z. B. Äthylmercaptan, Mercaptoessigsäure oder Triophenol in Frage. Besonders günstig erwies sich Äthylmercaptan, da es leicht flüchtig ist und sich gut mit Äther entfernen läßt. Vorzugsweise verwendet man Mischungen, die auf 1 Mol zu spaltende Substanz etwa 100 Mol oder mehr Mercaptan enthalten.

Die Trifluoressigsäurekonzentration sollte mindestens 30vol.-%ig sein, besser aber nicht unter 40–50 Vol.-% fallen. Die Reaktionszeit für die Tritylabspaltung liegt bei einer 50%igen Trifluoressigsäure-Äthylmercaptan-Lösung bei 10–60 Minuten, meist bei 30 Minuten. Zur Aufarbeitung wird zweckmäßig in Wasser eingetragen. Nach dem Eintragen in überschüssiges Wasser wird das Peptid je nach Löslichkeit entweder durch Absaugen oder durch Gefriertrocknung der mit Äther ausgeschüttelten wäßrigen Phase gewonnen.

Das von den Schutzgruppen befreite Peptid kann auch durch Ausfällen in organischen Lösungsmitteln, wie z. B. Diäthyläther, Diisopropyläther oder Essigester isoliert werden. Diese Aufarbeitungsmethode wird vorteilhaft dann angewendet, wenn sich die anfallenden Peptide schwer in Wasser lösen. Eventuell vorhandene leicht sauer abspaltbare Schutzgruppen wie z. B. der 3,5-Dimethoxy-, -dimethylbenzyloxycarbonyl-(Ddz)-Rest, Boc-Rest oder tert.-Butylester und -äther werden bei dem erfindungsgemäßen Verfahren ebenfalls mit abgespalten.

Je nach Art dieser Schutzgruppen muß die Reaktionszeit mehr oder weniger ausgedehnt werden. Für eine vollständige Abspaltung der Schutzgruppen vom tert.-Butyl-Typ sind in einer 50vol.-%igen Trifluoressigsäure-Äthylmercaptan-Lösung z. B. etwa 4 Stunden nötig. Bei längeren Peptiden mit mehreren ungeschützten Mercaptogruppen kann eine Wiederholung der Abspaltung vorteilhaft sein.

Als Reaktionsnebenprodukt entsteht z. B. bei der Tritylabspaltung mit Trifluoressigsäure/Äthylmercaptan S-Tritylmercaptoäthan, das bei der Aufarbeitung in die organische Phase geht.

Besonders wertvoll ist das erfindungsgemäße Verfahren bei höheren Peptiden, bei denen die Jodabspaltung aus Gründen der Löslichkeit oder Oxydationsempfindlichkeit nicht mehr möglich ist. Peptide, die in Methanol oder wäßriger Essigsäure nur noch schlecht löslich sind, lösen sich noch spielend in den erfindungsgemäßen Trifluoressigsäure-Mercaptan-Mischungen.

So konnte z. B. eine synthetisch aufgebaute geschützte Humaninsulin-A-Kette in etwa 70prozentiger Ausbeute durch zweimaliges Behandeln mit Mercaptoäthanol/Trifluoressigsäure zur Entfernung der 4 S-Tritylreste und der 7 tert.-Butylschutzgruppen gewonnen werden. Diese freie reduzierte Insulin-A-Kette wurde zur Charakterisierung in das Tetrasulfonat überführt. Es entstand ein Produkt, das sowohl in der sauren Elektrophorese als auch dünnschichtchromatographisch mit natürlicher Humaninsulin-A-Kette identisch war.

Die erhaltenen cysteinhaltigen Peptide sind entweder direkt therapeutisch verwendbar wie z. B. Somatostatin zur Behandlung der Pancreatitis oder dienen zur Synthese höherer, therapeutisch wertvoller Peptide.

Beispiel 1

a) H-Cys-Asn-OH-trifluoracetat

$a_1$) Zu einer Mischung von 25 ml Äthylmercaptan und 25 ml Trifluoressigsäure gibt man unter Rühren 5,34 g (10 mMol) H-Cys(Trt)-Asn-OBu$^t$ (Helv. Chim. Acta, 54, 398 [1971]). Man läßt 4 Stunden bei Raumtemperatur rühren und gibt die Mischung in 250 ml Wasser. Mit Äther wird nun dreimal extrahiert und die wäßrige Phase gefriergetrocknet. Ausb. 3,271 g (89%). $[\alpha]_D^{23} = +3,0°$ (c=1, in Methanol).

$C_7H_{13}N_3O_4S \cdot CF_3COOH \cdot H_2O$ (367,3)

Ber. C 29,40 H 4,39 N 11,44 S 8,73 $H_2O$ 4,9

Gef. C 30,5 H 4,0 N 11,2 S 8,9 $H_2O$ 4,6

$a_2$) Zu einer Mischung von 7,5 ml Äthylmercaptan und 7,5 ml Trifluoressigsäure gibt man unter Rühren 1,6 g (3 mMol) H-Cys(Trt)-Asn-OBu$^t$. Man läßt 4 Stunden bei Raumtemperatur stehen und schüttet die Mischung in 50 ml Diisopropyläther. Der Niederschlag wird abgesaugt und mit Diisopropyläther gewaschen und getrocknet. Ausb. 1 g (91%). $[\alpha]_D^{24} = +3,6°$ (c=1, in Wasser).

Die Substanz ist dünnschichtchromatographisch identisch mit unter $a_1$) gewonnener Substanz.

$a_3$) Ansatz analog Beispiel $a_2$. Statt Diisopropyläther wird Diäthyläther verwendet. Ausb. 0,95 g (86%). $[\alpha]_D^{23} = +3°$ (c=1, in Wasser).

Die Substanz ist dünnschichtchromatographisch identisch mit unter $a_1$ gewonnener Substanz.

$a_4$) Zu einer Mischung von 3,7 ml Merkaptoessigsäure und 3,7 ml Trifluoressigsäure gibt man unter Rühren 533,7 mg (1 mMol) H-Cys(Trt)-Ans-OBu$^t$. Man läßt 4 Stunden bei Raumtemperatur rühren und schüttet die Mischung in 50 ml Diäthyläther. Der Niederschlag wird abgesaugt und mit Diäthyläther gewaschen. Ausbeute 210 mg (57%).

$[\alpha]_D^{22} = 2,9°$ (c=1, in Wasser).

b) $(H\text{-}Cys\text{-}Asn\text{-}OH)_2$ (Jodoxydation von H-Cys-Asn-OH)

2,6 g (7 mMol) H-Cys-Asn-OH $\cdot$ $CF_3COOH \cdot H_2O$ werden in 25 ml 40proz. Essigsäure gelöst und mit 0,1 n-Jod-Lösung in Essigsäure titriert. Es werden 67,8 ml Jodlösung verbraucht (=97% d. Th.).

Danach wird eingeengt. Der Rückstand wird in Wasser gelöst und die wäßrige Lösung mit Äther ausgeschüttelt. Die wäßrige Phase wird gefriergetrocknet. Ausb. 2,42 g (97,6%).

$C_7H_{12}N_3O_4S \cdot 0,8\,HJ \cdot H_2O$ (354,6)

Ber. C 23,71 H 4,20 N 11,85 S 9,04 J 28,63

Gef. C 24,1 H 4,1 N 10,5 S 8,6 J 28,4

500 mg der oben gewonnenen Substanz wurden in wenig Wasser gelöst und über Amberlite IR 45 (Acetat-Form) (25 × 1,5 cm) chromatographiert. Das Eluat wird gefriergetrocknet. Ausb. 260,04 mg (67% d. Theorie, bezogen auf unoxydiertes Material). $[\alpha]_D^{25} = -62,1°$ (c=1, in Wasser).

$C_7H_{12}N_3O_4S \cdot 0,25\,CH_3COOH \cdot H_2O$ (268,3)

Ber. C 33,57 H 6,01 N 15,66 Essigs. 5,59 $H_2O$ 6,71

Gef. C 34,6 H 5,7 N 16,0 Essigs. 5,7 $H_2O$ 6,9

Beispiel 2

a) H-Leu-Glu-Asn-Tyr-Cys-Asn-OH-trifluoracetat

639,8 mg (0,5 mMol) H-Leu-Glu(OBu$^t$)-Asn-Tyr(Bu$^t$)-Cys-(Trt)-Asn-OBu$^t$ $\cdot$ $CF_3COOH$ werden in einer Mischung aus 2,5 ml Äthylmercaptan und 2,5 ml Trifluoressigsäure gelöst. Man läßt 4 Stunden bei Raumtemperatur rühren und gießt das Reaktionsgemisch in 50 ml Wasser. Die wäßrige Phase wird dreimal mit Äther extrahiert und gefriergetrocknet. Ausb. 331,5 mg (73%), $[\alpha]_D^{24} = -30,4°$ (c=1, in Wasser).

$C_{31}H_{46}N_8O_{12}S \cdot CF_3COOH \cdot 2\,H_2O$ (904,9)

Ber. C 43,80 H 5,45 N 12,38 S 3,54

Gef. C 44,4 H 5,6 N 12,3 S 3,8

b) Herstellung der Ausgangssubstanz
H-Leu-Glu(OBu$^t$)-Asn-Tyr(Bu$^t$)-Cys(Trt)-Asn-OBu$^t$-trifluoracetat
$b_1$) Ddz-Leu-OTcp

33,3 g Ddz-Leu-OH (Ann. Chem., 1973, 1652–1662) (94,3 mMol) werden mit 19,6 g (0,1 Mol) 2,4,5-Trichlorphenol in 350 ml Essigester gelöst. Dazu gibt man bei 0° C eine Lösung von 20,4 g DCC in 60 ml Essigester. Man läßt 3 Stunden bei 0° und über Nacht bei Raumtemperatur rühren. Der ausgefallene Dicyclohexylharnstoff wird abgesaugt und das Filtrat eingeengt. Der Rückstand kristallisiert mit Petroläther über Nacht im Kühlraum. Ausb. 39,2 g (73,6%, bezogen auf H-Leu-OMe · HCl). Schmp. 108°, $[\alpha]_D^{29} = -31{,}3°$ (c=1, in Dimethylacetamid).

$C_{24}H_{28}NO_6Cl_3$ (532,83)
Ber. C 54,10 H 5,30 N 2,62 Cl 20,01
Gef. C 54,7 H 5,4 N 2,9 Cl 19,3

$b_2$) Ddz-Leu-Glu(OBu$^t$)-Asn-Tyr(Bu$^t$)-OH · 0,5 $H_2O$

27,7 g (50 mMol) H-Glu(OBu$^t$)-Asn-Tyr(Bu$^t$)-OH (Chem. Ber., 110, 1–11 [1977]) werden in 150 ml Dimethylformamid suspendiert. Nach Zugabe von 6,75 g HOBt (50 mMol) tritt Lösung ein. Man kühlt auf 0° C ab, gibt 6,5 ml (50 mMol) N-Äthylmorpholin und 28 g (52,5 mMol) Ddz-Leu-OTcp zu. Es wurde 1 Std. bei 0° C und 3 Std. bei Raumtemperatur gerührt. Eine Fluoramprobe ergab, daß noch nicht die ganze Aminokomponente abreagiert war. Man gibt nun nochmals 1,3 g Ddz-Leu-OTcp (2,5 mMol) zu und läßt einige Stunden bei Raumtemperatur reagieren. Ist der Ansatz Fluoram negativ, wird aufgearbeitet. Das Lösungsmittel wird im Hochvakuum abdestilliert und der Rückstand in Essigester gelöst. Es wird mit eiskaltem Citratpuffer (pH 3) auf pH 4 eingestellt. Anschließend wird die Essigesterphase einmal mit Citratpuffer (pH 4) und zweimal mit Wasser gewaschen. Man trocknet über $Na_2SO_4$ und engt ein. Der Rückstand wird mit einer Mischung aus Äther und Petroläther wie 1 : 1 verrieben. Man stellt einige Stunden in den Kühlraum und saugt ab.
Ausb. 35,35 g (80,25%), Schmp. 222–223°, $[\alpha]_D^{25} = -23{,}7°$ (c=1, in Methanol).

$C_{44}H_{65}N_5O_{13}$ · 0,5 $H_2O$ (881,04)
Ber. C 60,00 H 7,55 N 7,95
Gef. C 60,0 H 7,6 N 8,0

$b_3$) Ddz-Lei-Glu(OBu$^t$)-Asn-Tyr(Bu$^t$)-Cys(Trt)-Asn-OBu$^t$

Zu einer auf 0° C abgekühlten Lösung von 26,4 g (30 mMol) Ddz-Leu-Glu(OBu$^t$)-Asn-Tyr(Bu$^t$)-OH-hemihydrat, 16,0 g (30 mMol) H-Cys(Trt)-Asn-OBu$^t$ und 4,05 g (30 mMol) HOBt in 60 ml Dimethylformamid gibt man eine auf 0° C abgekühlte Lösung von 6,6 g DCC in 20 ml Dimethylformamid. Man läßt den Ansatz 2 Stunden bei 0° C und über Nacht bei Raumtemperatur rühren. Der Dicyclohexylharnstoff wird abgesaugt und mit 20 ml Dimethylformamid gewaschen. Das Filtrat gießt man unter Rühren vorsichtig in ca. 250 ml gekühlte $NaHCO_3$-Lösung und extrahiert mit 600 ml Essigester. Die Essigesterphase wird nacheinander 1 × mit 300 ml $NaHCO_3$-Lösung, 1 × 300 ml Citratpuffer (pH 4) und 1 × mit 100 ml $NaHCO_3$-Lösung ausgeschüttelt, über $Na_2SO_4$ getrocknet und eingeengt. Der Rückstand wird mit Äther verrieben. Ausb. 28–34 g (67–81%). Schmp. 161–165°, $[\alpha]_D^{23} = -13{,}1°$ (c=1, in $CH_2Cl_2$).

$C_{74}H_{98}N_8O_{16}S$ (1387,73)
Ber. C 64,05 H 7,12 N 8,07 S 2,31
Gef. C 63,8 H 7,3 N 8,0 S 2,7

$b_4$) H-Leu-Glu(OBu$^t$)-Asn-Tyr(Bu$^t$)-Cys(Trt)-Asn-OBu$^t$-trifluoracetat

27,75 g (10 mMol) Ddz-Leu-Glu(OBu$^t$)-Asn-Tyr(Bu$^t$)-Cys-(Trt)-Asn-OBu$^t$ werden bei Raumtemperatur in einer Mischung aus 17,5 ml Trifluoressigsäure (0,2 Mol), 3,5 ml Wasser und 330 ml Methylenchlorid (= ca. 350 ml 5%ige Trifluoressigsäure mit 1% Wasser in Methylenchlorid) eingetragen. Man läßt 2 Stunden bei Raumtemperatur rühren. Darauf kühlt man auf 0° C ab, neutralisiert die Trifluoressigsäure mit 17,75 ml (220 mMol) Pyridin und engt im Vakuum ein. Der Rückstand wird in 250 ml Essigester gelöst und 3mal mit 25 ml Wasser ausgeschüttelt. Die Essigesterphase wird ohne zuvor getrocknet zu werden, eingeengt. Der Rückstand wird im Hochvakuum getrocknet und mit Äther verrieben. (Statt Diäthyläther kann auch Diisopropyläther verwendet werden.) Der Niederschlag wird abgesaugt, mit Äther gewaschen und getrocknet. Ausb. 25,2 g. Zur weiteren Reinigung werden ca. 6-g-Portionen in 30 ml Essigester zum Sieden erhitzt und warm zentrifugiert. Die Niederschläge werden je 2mal mit Äther verrührt, zentrifugiert und am Hochvakuum über $P_2O_5$ getrocknet. Ausb. 22,3 g (86%). Schmp. 178–182°, 161–164°, 166–171°, $[\alpha]_D^{22} = -3{,}5$ bis $+4{,}0°$ (c=1, in 90proz. Essigsäure).

$C_{64}H_{85}N_8O_{14}SF_3$ (1279,5)
Ber. C 60,08 H 6,70 N 8,76 S 2,51
Gef. C 59,4 H 6,5 N 9,1 S 2,9

Aminosäureanalyse: Asp (2,0), Glu (1,05), Cys (0,87), Leu (0,99), Tyr (1,05).

Beispiel 3
a) H-Tyr-Gln-Leu-Glu-Asn-Tyr-Cys-Asn-OH-trifluoracetat

814 mg (0,5 mMol) H-Tyr($Bu^t$)-Gln-Leu-Glu($OBu^t$)-Asn-Tyr($Bu^t$)-Cys(Trt)-Asn-$OBu^t$-trifluoracetat werden in einer Mischung aus 5 ml Äthylmercaptan und 5 ml Trifluoressigsäure gelöst. Man läßt 4 Stunden bei Raumtemperatur stehen und gießt das Reaktionsgemisch in 50 ml Wasser. Die wäßrige Phase wird dreimal mit Äther extrahiert und gefriergetrocknet. Ausb. 590 mg (98%). $[\alpha]_D^{25} = -15,3°$ (c=1, in 90proz. Essigsäure).

$C_{45}H_{63}N_{11}O_{16}S \cdot CF_3COOH \cdot 2\,H_2O$ (1196,2)
Ber. C 47,19 H 5,73 N 12,88 S 2,68
Gef. C 47,0 H 5,6 N 12,0 S 3,0

b) Herstellung der Ausgangssubstanz
H-Tyr($Bu^t$)-Gln-Leu-Glu($OBu^t$)-Asn-Tyr($Bu^t$)-Cys(Trt)-Asn-$OBu^t$-trifluoracetat
$b_1$) H-Tyr($Bu^t$)-OMe · HCl

300 g (778 mMol) Z-Tyr($Bu^t$)-OMe (Ann. Chem., 696, 226 [1966]) werden in 1 l Methanol gelöst und an der Autobürette (Zugabe von ca 2 n-methanolischer HCl) bei pH 4,5 katalytisch (Pd/$BaSO_4$)-Katalysator) hydriert.

Nach beendigter Hydrierung wird der Katalysator abgesaugt und das Filtrat eingeengt. Der Rückstand wird mit Äther verrieben. Ausb. 189,2 g (84,5%). Schmp. 154–156°, $[\alpha]_D^{22} = +15,0°$ (c=1, in Methanol).

$C_{14}H_{22}NO_3Cl$ (287,79)
Ber. C 58,43 H 7,7 N 4,86
Gef. C 58,6 H 7,6 N 4,8

$b_2$) Ddz-Tyr($Bu^t$)-OMe

Zu einer Lösung von 71,95 g (0,25 Mol) H-Tyr($Bu^t$)-OMe · HCl in 110 ml Dimethylformamid gibt man unter Eiskühlung und Rühren 35 ml (0,25 Mol) Triäthylamin und 79,5 g (0,3 Mol) Ddz-azid. Nach 5 Minuten gibt man weitere 35 ml (0,25 Mol) Triäthylamin zu. Nun entfernt man das Eisbad und läßt auf Raumtemperatur kommen. Nach 30 Minuten gibt man 7 ml (50 mMol) Triäthylamin zu und läßt 3 Stunden bei Raumtemperatur rühren. Der Ansatz bleibt über Nacht bei Raumtemperatur stehen, und wird zwischen 250 ml Essigester und 250 ml Wasser verteilt. Die Essigesterphase wird nach Zugabe von Eis 3mal mit je 125 ml eiskaltem Citratpuffer (pH 3), 1mal mit 250 ml ges. $NaHCO_3$-Lösung und 1mal mit 125 ml Wasser gewaschen, über $Na_2SO_4$ getrocknet und eingeengt. Am Hochvakuum wird getrocknet. Ausb. 118,2 g Öl (99,8%).

$b_3$) Ddz-Tyr($Bu^t$)-OH

Die unter 2) gewonnenen 118,2 g (249,5 mMol) öligen Ddz-Tyr($Bu^t$)-OMe werden in 500 ml 1,2-Dimethoxyäthan gelöst und mit einer Spatelspitze Thymolphthalein versetzt. Dazu läßt man unter Rühren 250 ml 1 n-NaOH zutropfen. Man rührt, bis die blaue Farbe des Ansatzes verblaßt ist. Nun werden nach und nach in mehreren Portionen noch 120 ml 1 n-NaOH zugesetzt, bis sich die Lösung nicht mehr entfärbt. (Der Mehrverbrauch an Natronlauge erklärt sich durch den Essigestergehalt des Ausgangsproduktes.) Danach wird mit Citronensäure neutralisiert und die Lösung eingeengt. Der Rückstand wird mit Eiswasser versetzt und bei 0°C mit Citronensäure auf pH 3,5 gestellt. Das entstehende Öl wird in Essigester aufgenommen. Die Essigesterphase wird zweimal mit 200 ml Citratpuffer (pH 4) und zweimal mit 200 ml Wasser ausgeschüttelt. Die Essigesterphase wird über $Na_2SO_4$ getrocknet, abdestilliert und am Hochvakuum getrocknet. Es bleibt eine gelblichweiße amorphe Substanz zurück. Ausb. 99,5 g (86,8%). Schmp. ca. 38–40°, $[\alpha]_D^{22} = +41,5°$ (c=1, in Methylenchlorid).

$C_{25}H_{33}NO_7$ (459,55)
Ber. C 65,34 H 7,24 N 3,05
Gef.. C 65,0 H 7,3 N 3,0

b$_4$) Ddz-Tyr(Bu$^t$)-Gln-ONb

Zu einer Lösung von 25,24 g (55 mmol) Ddz-Tyr(Bu$^t$)-OH, 15,88 g (50 mmol) H-Gln-ONb · HCl (Chem. Ber. 110, 1—11 [1977]) und 6,75 g HOBt-monohydrat in 100 ml Dimethylformamid gibt man bei −3°C 6,4 ml (50 mmol) N-Äthylmorpholin und 10,5 g DCC. Man läßt 1 Std. bei 0°C und 6 Std. bei Raumtemperatur rühren und über Nacht bei Raumtemperatur stehen. Der Niederschlag wird abgesaugt und das Filtrat eingeengt. Das resultierende Öl wird in Essigester gelöst und die Lösung nacheinander mit $NaHCO_3$-Lösung, Citratpuffer (pH 3) und Wasser gewaschen, über $Na_2SO_4$ getrocknet und eingeengt. Das Öl wird mit Petroläther pulvrig verrieben und abgesaugt. Zur weiteren Reinigung wird die Substanz dreimal mit je 100 ml Diisopropyläther gekocht und abdekantiert. Zum Schluß wird mit kaltem Diisopropyläther verrieben, abgesaugt und mit Petroläther nachgewaschen.
Ausb. 32,8 g (91%), Schmp. 80—90°, $[\alpha]_D^{22} = +15,1°$ (c = 1, in Methanol).

$C_{37}H_{46}N_4O_{11}$ (722,81)
Ber. C 61,48 H 6,41 N 7,75
Gef. C 61,3 H 6,7 N 7,9

b$_5$) Ddz-Tyr(Bu$^t$)-Gln-OH-DCHA

Zu einer Lösung von 32,5 g (45 mmol) Ddz-Tyr(Bu$^t$)-Gln-ONb in 500 ml Methanol gibt man 5 ml Wasser und Pd/$BaSO_4$-Katalysator und hydriert 7 Stunden. Danach wird vom Katalysator abgesaugt und das Filtrat eingeengt. Das zurückbleibende Öl wird in 250 ml Essigester gelöst. Dazu gibt man 11,3 ml (55 mmol) Dicyclohexylamin. Man läßt einige Stunden im Kühlraum stehen und saugt den Niederschlag ab. Der Filterrückstand wird mit Essigester in der Reibschale verrieben, abgesaugt und im Vakuum getrocknet.
Ausb. 27 g (78%).
Schmp. 170—171°, $[\alpha]_D^{23} = +10,2°$ (c = 1, in Methanol).

$C_{42}H_{64}N_4O_9$ (769,0)
Ber. C 65,6 H 8,39 N 7,28
Gef. C 65,4 H 8,5 N 7,3

b$_6$ Ddz-Tyr(Bu$^t$)-Gln-OH

2,9 g (3,77 mmol) Ddz-Tyr(Bu$^t$)-Gln-OH · DCHA werden zwischen Essigester und Citratpuffer (pH 3) verteilt. Die Essigesterphase wird mit Wasser neutral gewaschen, über $Na_2SO_4$ getrocknet und eingeengt. Es bleibt ein amorphes Produkt zurück.
Ausb. 2 g (90%).
Schmp. 110—115°, $[\alpha]_D^{22} = +19,8°$ (c = 1, in Methanol).

$C_{30}H_{41}N_3O_9$ (587,68)
Ber. C 61,31 H 7,03 N 7,15
Gef. C 60,6 H 7,2 N 7,0

b$_7$) Ddz-Tyr(Bu$^t$)-Gln-Leu-Glu(OBu$^t$)-Asn-Tyr(Bu$^t$)-Cys(Trt)-Asn-OBu$^t$

9,7 g (16,5 mmol) Ddz-Tyr(Bu$^t$)-Gln-OH, 19,2 g (15 mmol) H-Leu-Glu(OBu$^t$)-Asn-Tyr(Bu$^t$)-Cys(Trt)-Asn-OBu$^t$ · $CF_3COOH$ und 2,025 g (15 mmol) HOBt werden in 30 ml Dimethylformamid unter Rühren bei Raumtemperatur gelöst. Man kühlt dann auf 0°C ab und versetzt mit 1,95 ml (15 mmol) N-Äthylmorpholin und einer Lösung von 3,3 g (16 mmol) DCC in 9 ml Dimethylformamid. Man läßt 1 Stunde bei 0° und 4 Stunden bei Raumtemperatur rühren, läßt über Nacht bei Raumtemperatur stehen und saugt den Dicyclohexylharnstoff ab. Mit je 4,5 ml Dimethylformamid wird zweimal nachgewaschen. Das Filtrat läßt man unter Rühren zu 159 ml gesättigter $NaHCO_3$-Lösung einfließen und rührt so lange, bis ein pulvriger Niederschlag entsteht. Der Niederschlag wird abgesaugt, mit Citratpuffer (pH 3) verrieben, abgesaugt und mit Wasser neutral gewaschen. Der Niederschlag wird im Hochvakuum getrocknet.

Ausb. 23,1 g.

Die Rohsubstanz wird auf dem Dampfbad bis fast zum Sieden erwärmt. Die dünne Suspension wird über Nacht in den Kühlraum gestellt und der Niederschlag abgesaugt. Mit Essigester und Äther wird gewaschen.

Ausb. 20 g (76,8%).

$[\alpha]_D^{24} = -10,2°$ (c = 1, in Methanol). Die Suspension zersetzt sich ab 205° und verkohlt bei ca. 250°.

$C_{92}H_{123}N_{11}O_{20}S$ (1735,15)
Aminosäureanalyse:
Ber. Asp 2,0 Glu 2,0 Cys 1,0 Leu 1,0 Tyr 2,0
Gef. Asp 2,00 Glu 2,01 Cys 0,75 Leu 0,99 Tyr 1,95

$b_8$) H-Tyr($Bu^t$)-Gln-Leu-Glu($OBu^t$)-Asn-Tyr($Bu^t$)-Cys(Trt)-Asn-$OBu^t$-trifluoracetat

3,5 g (2 mmol) Ddz-Tyr($Bu^t$)-Gln-Leu-Glu($OBu^t$)-Asn-Tyr($Bu^t$)-Cys(Trt)-Asn-$OBu^t$ werden in einer Mischung aus 1,75 ml Trifluoressigsäure (20 mmol), 0,35 ml Wasser und 33 ml Methylenchlorid (= ca. 35 ml einer 5%igen Trifluoressigsäurelösung mit 1% Wasser) und 3,5 ml Anisol unter Rühren gelöst.

Man läßt 3 Stunden bei Raumtemperatur rühren, versetzt anschließend mit 2 ml (24,8 mmol) Pyridin und engt im Hochvakuum ein. Der Rückstand wird mit Äther verrieben, abgesaugt, mit Wasser gewaschen und über $P_2O_5$ getrocknet.

Ausb. 3,35 g.

Zur weiteren Reinigung wurde die Substanz mit je 20 ml Essigester kurz zum Sieden erhitzt und heiß abgesaugt. Mit Äther wurde nachgewaschen.

Ausb. 3,0 g (92%), Schmp. 255—265° (Zers.), $[\alpha]_D^{24} = -23,8°$ (c = 1, in Methanol).

$C_{80}H_{109}N_{11}={}_{16}S \cdot CF_3COOH$ (1626,9)
Aminosäureanalyse:
Ber. Asp 2,0 Glu 2,0 Cys 1,0 Leu 1,0 Tyr 2,0
Gef. Asp 1,95 Glu 2,08 Cys 0,81 Leu 1,07 Tyr 2,04

## Beispiel 4

### a) H-Cys-Ser-Leu-OH-trifluoracetat

564 mg (0,97 mmol) H-Cys(Trt)-Ser-Leu-OH · $H_2O$ (Chem. Ber. 103, 2034 [1970]) werden in einer Mischung aus 2,5 ml Äthylmercaptan und 2,5 ml Trifluoressigsäure gelöst. Man läßt 30 Minuten bei Raumtemperatur stehen und schüttet den Ansatz in 50 ml Wasser. Die wäßrige Phase wird dreimal ausgeäthert und gefriergetrocknet.

Ausb. 331 mg (78%).

$[\alpha]_D^{28} = -15,4°$ (C = 1, in Methanol)

$C_{12}H_{21}N_3O_4S \cdot CF_3COOH \cdot H_2O$ (435,4)
Ber. C 38,62 H 5,56 N 9,65
Gef. C 38,7 H 6,0 N 9,4

Das gleiche Ergebnis wie oben wird erhalten, wenn 564 mg H-Cys(Trt)Ser-Leu-OH · $H_2O$ in einer Mischung aus 11 ml Trifluoressigsäure und 11 ml Thiophenol gespalten werden (sonst gleiche Bedingungen wie oben).

## Beispiel 5

### a) H-Ile-Cys-Ser-Leu-OH-trifluoracetat

677 mg (0,91 mmol) H-Ile-Cys(Trt)-Ser-Leu-OH · 0,3 $CF_3$-COOH · 1,5 $H_2O$ werden in einer Mischung aus 2,5 ml Äthylmercaptan und 2,5 ml Trifluoressigsäure gelöst. Man läßt 30 Minuten bei Raumtemperatur rühren und gießt das Reaktionsgemisch unter Rühren in 50 ml Wasser. Die wäßrige Phase wird dreimal mit Äther extrahiert und anschließend gefriergetrocknet.

Ausb. 506 mg (98%).

$[\alpha]_D^{24} = -11,3°$ (c = 1, in Methanol).

$C_{18}H_{34}N_4O_6S \cdot CF_3COOH \cdot H_2O$ (566,6)
Ber. C 42,39 H 6,58 N 9,89
Gef. C 42,4 H 7,0 N 10,6

b) Herstellung der Ausgangssubstanz H-Ile-Cys(Trt)-Ser-Leu-OH
$b_1$) Ddz-Ile-OTcp

Zu einer auf 0° C gekühlten Lösung von 87 g (246 mmol) Ddz-Ile-OH (Ann. Chem. 763, 162—172 [1972]) und 48,6 g (246 mmol) 2,4,5-Trichlorphenol in 700 ml Essigester gibt man eine kalte Lösung von 52 g (252 mmol) DCC in 175 ml Essigester. Man läßt 3 Stunden bei 0°C rühren und über Nacht bei Raumtemperatur stehen. Der Dicyclohexylharnstoff wird abgesaugt und das Filtrat im Vakuum eingeengt. Der Rückstand kristallisiert mit Petroläther.

Ausb. 105,6 g (80,5%).

Schmp. 92—94°, $[\alpha]_D^{22}$ = −22,6° (c = 1, in Methanol).

$C_{24}H_{28}NO_6Cl_3$ (532,86)

| | | | | |
|---|---|---|---|---|
| Ber. | C 54,10 | H 5,30 | N 2,63 | Cl 19,96 |
| Gef. | C 54,4 | H 5,4 | N 2,8 | Cl 19,5 |

$b_2$) Ddz-Ile-Cys(Trt)-Ser-Leu-OH · 1 $H_2O$

Zu einer Lösung von 69,8 g (120 mmol) H-Cys(Trt)-Ser-Leu-OH-monohydrat (Chem. Ber. 103, 2034—2040 [1970]) und 16,2 g (120 mmol) HOBt in 300 ml Dimethylformamid gibt man bei Raumtemperatur 63,95 g (120 mmol) Ddz-Ile-OTcp und rührt 4 Stunden bei Raumtemperatur. Danach gibt man nochmals 5,33 g (10 mmol) Ddz-Ile-OTcp zu wenn Tripeptid noch vorhanden ist. Man läßt über Nacht bei Raumtemperatur stehen und destilliert das Lösungsmittel im Vakuum ab. Das zurückbleibende Öl wird zwischen 400 ml Essigester und 250 ml gesättigter Natriumcarbonatlösung verteilt, wobei das Natriumsalz des Ddz-Tetrapeptids im Essigester bleibt. Die Essigesterphase wird gekühlt und nacheinander mit ca. 120 ml 1n Citronensäurelösung (wäßriger Phase sollte nach dem Ausschütteln etwa pH 3—4 haben), 150 ml Citratpuffer (pH 3) und 2mal mit 200 ml Wasser ausgeschüttelt. Danach wird die Essigesterphase über $Na_2SO_4$ getrocknet und im Vakuum eingeengt. Der Rückstand wird mit Petroläther verrieben und abgesaugt.

Ausb. 99,8 g (90%).

Schmp. (130) 135—140° (Zers.),

$[\alpha]_D^{22}$ = −34,1° (c = 1, in Methanol).

$C_{49}H_{62}N_4O_{10}S$ · 1 $H_2O$ (917,1)

| | | | | |
|---|---|---|---|---|
| Ber. | C 62,93 | H 7,11 | N 6,02 | S 3,42 |
| Gef. | C 63,0 | H 6,8 | N 5,8 | S 3,4 |

$b_3$) H-Ile-Cys(Trt)-Ser-Leu-OH

2,7 g (2,94 mmol) Ddz-Ile-Cys(Trt)-Ser-Leu-OH werden in 52 ml einer 5proz. Trifluoressigsäure-Methylenchloridlösung mit 1% Wasser gelöst und läßt 4 Stunden bei Raumtemperatur rühren. Danach wird mit 3 ml Pyridin neutralisiert und die Lösung i. Vak. eingeengt. Der Rückstand wird mit Wasser verrieben, abgesaugt, mit Wasser gewaschen und über $P_2O_5$ getrocknet. Zur weiteren Reinigung wird die Substanz zweimal mit jeweils 30 ml Essigester ausgekocht und heiß abgesaugt.

Ausb. 1,51 g (70%).

$[\alpha]_D^{22}$ = +3,7° (c = 1, in 90proz. Essigsäure).

$C_{37}H_{48}N_4O_6S$ · 0,3 $CF_3COOH$ · 1,5 $H_2O$ (738,1)

| | | | | |
|---|---|---|---|---|
| Ber. | C 61,18 | H 7,00 | N 7,59 | $CF_3COOH$ 4,63 |
| Gef. | C 61,0 | H 7,0 | N 7,7 | $CF_3COOH$ 4,7 |

## Beispiel 6

a) H-Ile-Cys-Ser-Leu-Tyr-Gln-Leu-Glu-Asn-Tyr-Cys-Asn-OH

3,429 g (1,5 mmol) H-Ile-Cys(Trt)-Ser-Leu-Tyr($Bu^t$)-Gln-Leu-Glu($OBu^t$)-Asn-Tyr($Bu^t$)-Cys(Trt)-Asn-$OBu^t$-trifluoracetat werden in einer Mischung aus 19 ml Äthylmercaptan und 19 ml Trifluoressigsäure gelöst. Man läßt 4 Stunden bei Raumtemperatur rühren und gießt das Reaktionsgemisch unter Rühren in 250 ml Wasser. Der Niederschlag wird abgesaugt, mit Äther gewaschen und getrocknet. Anschließend wird mit Äther verrieben, abgesaugt und getrocknet. Ausb. 1,964 g. Diese Substanz wird noch einmal wie oben gespalten. Ausbeute 1,6 g (61%). Aus den Filtraten konnte nach Extraktion mit Äther und Gefriertrocknung noch insgesamt 330 mg Substanz gewonnen werden.

Gesamtausbeute: 1,93 g (73%).

$C_{63}H_{95}N_{15}O_{21}S_2 \cdot CF_3COOH \cdot 10\ H_2O$ (1756,9)
Ber. C 44,43 H 6,65 N 11,96 S 3,65
Gef. C 44,1 H 6,0 N 11,6 S 4,3

Das NMR-Spektrum zeigt keine $Bu^t$-Signale und nur eine Spur (ca. 5%) des Triphenylmethylsignals. Die 18 Protonen der Methylsignale von Isoleucin und den beiden Leucinen bei $\delta = 0{,}7-1{,}0$ stehen im richtigen Verhältnis zu den 8 aromatischen Protonen der Tyrosine bei $\delta = 6{,}5-7{,}1$ ppm.

b) Herstellung der Ausgangssubstanz
H-Ile-Cys(Trt)-Ser-Leu-Tyr($Bu^t$)-Gln-Leu-Glu($OBu^t$)-Asn-Tyr($Bu^t$)-Cys(Trt)-Asn-$OBu^t$-trifluoracetat

$b_1$) Ddz-Ile-Cys(Trt)-Ser-Leu-Tyr($Bu^t$)-Gln-Leu-Glu($OBu^t$)-Asn-Tyr($Bu^t$)-Cys(Trt)-Asn-$OBu^t$

Zu einer Lösung von 22,8 g (14 mmol) H-Tyr($Bu^t$)-Gln-Leu-Glu($OBu^t$)-Asn-Tyr-$Bu^t$)-Cys(Trt)-Asn-$OBu^t$, 14,1 g (15,4 mmol) Ddz-Ile-Cys(Trt)-Ser-Leu-OH · 1 $H_2O$ und 2,08 g (15,4 mmol) HOBt und 1,8 ml (14 mmol) N-Äthylmorpholin in 70 ml Dimethylformamid gibt man bei 0° C 3,4 g (16,4 mmol) DCCI. Man läßt 1 Stunde bei 0° C und 3 Stunden bei Raumtemperatur rühren, läßt über Nacht bei Raumtemperatur stehen und saugt den Niederschlag ab. Das Filtrat wird im Vakuum eingeengt und der Rückstand nacheinander mit $NaHCO_3$-Lösung, Citratpuffer (pH 3), $NaHCO_3$-Lösung und Wasser verrieben und jeweils abgesaugt. Über $P_2O_5$ wird getrocknet.
Ausbeute 33,18 g (99%).
Zur weiteren Reinigung wird die trockene Substanz in 300 ml warmen Essigester suspendiert und kurz auf dem Dampfbad erwärmt. Nach Zugabe von 300 ml Äther wird noch warm abgesaugt. Mit Äther wird nachgewaschen und am Hochvakuum getrocknet.
Ausbeute 28,1 g (84%), Schmp. 265—270° (Zers.).
$[\alpha]_D^{22} = -20{,}3°$ (c = 1, in Dimethylformamid).
Anschließend wurde noch mit 100 ml Methanol erhitzt und nach Stehen im Kühlraum (über Nacht) abgesaugt und getrocknet.
Ausbeute 23,75 g (71%).
$[\alpha]_D^{22} = -25{,}4°$ (c = 1, in Dimethylformamid).

$C_{129}H_{169}N_{15}O_{25}S_2$ (2394,0)
Aminosäureanalyse:
Ber. Asp 2,0 Ser 1,0 Glu 2,0 Cys 2,0 Ile 1,0 Leu 2,0 Tyr 2,0
Gef. Asp 2,10 Ser 0,95 Glu 1,77 Cys 0,49 Ile 0,83 Leu 2,10 Tyr 1,98

$b_2$) H-Ile-Cys(Trt)-Ser-Leu-Tyr($Bu^t$)-Gln-Leu-Glu($OBu^t$)-Asn-Tyr($Bu^t$)-Cys(Trt)-Asn-$OBu^t$-trifluoracetat

24 g (ca. 30 mmol) Ddz-Ile-Cys(Trt)-Ser-Leu-Tyr($Bu^t$)-Gln-Leu-Glu($Bu^t$)-Asn-Tyr($Bu^t$)-Cys(Trt)-Asn-$OBu^t$ werden in einer Mischung aus 8,75 ml (100 mmol) Trifluoressigsäure, 1,75 ml Wasser und 165 ml Methylenchlorid (= ca. 175 ml einer 5%igen Trifluoressigsäure-Methylenchlorid-Lösung mit 1% Wasser) und 17,5 ml Anisol unter Rühren gelöst. Man läßt 4 Stunden bei Raumtemperatur rühren. Danach versetzt man mit 10 ml (124 mmol) Pyridin und engt ein. Der Rückstand wird mit Äther verrieben, abgesaugt und mit Äther gewaschen und im Vakuum getrocknet. Danach wird die Substanz mit Wasser verrieben, abgesaugt, mit Wasser gewaschen und über $P_2O_5$ getrocknet.
Zur weiteren Reinigung wird die Substanz 2mal mit je 100 ml Essigester ausgekocht und jeweils heiß abgesaugt.
Ausbeute 22,7 g (99%), Schmp. 234° (Zers.),
$[\alpha]_D^{26} = -11{,}7°$ (c = 1, in Dimethylformamid).

$C_{117}H_{155}N_{15}O_{21}S_2 \cdot CF_3COOH$ (2285,8)
Aminosäureanalyse:
Ber. Asp 2,0 Ser 1,0 Glu 2,0 Cys 2,0 Ile 1,0 Leu 2,0 Tyr 2,0
Gef. Asp 2,05 Ser 0,99 Glu 1,88 Cys 0,43 Ile 0,73 Leu 2,12 Tyr 2,05

Beispiel 7

a) H-Cys-Cys-Thr-Ser-OH-trifluoracetat

615,8 mg (0,5 mmol) Ddz-Cys(Trt)-Cys(Trt)-Thr($Bu^t$)-Ser($Bu^t$)-OH werden in einer Mischung aus 2,5 ml Äthylmercaptan und 2,5 ml Trifluoressigsäure gelöst. Man läßt 4 Stunden bei Raumtemperatur rühren und gießt das Reaktionsgemisch in 50 ml Wasser. Die wäßrige Phase wird dreimal mit Äther extrahiert und gefriergetrocknet. Ausbeute 240 mg (88%), $[\alpha]_D^{24} = -24,3°$ (c = 1, in Wasser).

$C_{13}H_{24}N_4O_7S_2 \cdot CF_3COOH \cdot H_2O$ (544,5)
Ber. C 33,08 H 5,00 N 10,29 S 11,77
Gef. C 34,4 H 5,1 N 10,0 S 11,1

b) Herstellung der Ausgangssubstanz Ddz-Cys(Trt)-Cys(Trt)-Thr($Bu^t$)-Ser($Bu^t$)-OH

$b_1$) Z-Thr($Bu^t$)-Ser($Bu^t$)-OH

Zu einer Suspension von 16,1 g (0,1 mol) H-Ser($Bu^t$)-OH (Chem. Ber. 97, 2490 [1964]) in 100 ml Dimethylformamid gibt man bei 0°C 12,8 ml (0,1 mol) N-Äthylmorpholin und 40,6 g (0,1 mol) Z-Thr($Bu^t$)-OSu (Hoppe Seyler's Z. Physiol. Chem. 346, 60 [1966]).
Man rührt anschließend 5 Stunden bei Raumtemperatur und läßt über Nacht bei Raumtemperatur stehen.
DC-Kontrolle auf H-Ser($Bu^t$)-OH.
Ist kein H-Ser($Bu^t$)-OH dünnschichtchromatographisch feststellbar, wird von gegebenenfalls Ungelöstem abgesaugt. Das Filtrat wird eingeengt und der Rückstand zwischen Äther und 110 ml 1n Zitronensäure verteilt. Die ätherische Phase wird noch einmal mit 100 ml 0,5n Zitronensäure und anschließend mit Wasser oder NaCl-Lösung neutral gewaschen. Die ätherische Phase wird über $Na_2SO_4$ getrocknet, eingeengt und am Hochvakuum getrocknet. Ausbeute 32,5 g amorphes Produkt (72%).
$[\alpha]_D^{22} = +24,1°$ (c = 1, in Methanol).

$C_{22}H_{36}N_2O_7$ (452,55)
Ber. C 61,04 H 8,02 N 6,19
Gef. C 60,3 H 7,4 N 6,2

$b_2$) H-Thr($Bu^t$)-Ser($Bu^t$)-OH · HCl

25 g (55,24 mmol) Z-Thr($Bu^t$)-Ser($Bu^t$)-OH werden in 200 ml Methanol gelöst und am Autotitrator unter Zugabe von Methanol · HCl katalytisch hydriert (pH 4,5). Nach beendigter Hydrierung wird vom Pd/$BaSO_4$-Katalysator abgesaugt und das Filtrat eingeengt und am Hochvakuum getrocknet. Ausbeute 1,1 g amorphes Produkt (19,60 = 100%). $[\alpha]_D^{27} = +27°$ (c = 1, in Methanol).

$C_{15}H_{31}N_2O_5Cl$ (354,9)
Ber. C 50,77 H 8,80 N 7,89
Gef. C 50,9 H 8,8 N 7,8

$b_3$) Ddz-Cys(Trt)-OH

Zu einer Suspension von 36,4 g (0,1 mol) H-Cys(Trt)-OH (J. Org. Chem. 30, 1340 [1965]) in 200 ml Dimethylformamid gibt man 28 ml (0,2 mol) Triäthylamin und 26,5 g (0,1 mol) Ddz-Azid. Es wird 24 Stunden bei 40°C gerührt. Danach wird die Lösung eingeengt und der Rückstand zwischen 100 ml eisgekühlter 1n Zitronensäure und Essigester verteilt. Die Essigesterphase wird mit Zitratpuffer (pH 3) und NaCl-Lösung ausgeschüttelt, über $Na_2SO_4$ getrocknet und eingeengt. Am Hochvakuum wird getrocknet. Ausbeute 37,6 g amorphes Produkt (64%).
$[\alpha]_D^{22} = +27,0°$ (c = 1, in Methanol)

$C_{34}H_{35}NO_6S$ (585,7)
Ber. C 69,72 H 6,02 N 2,39
Gef. C 69,3 H 6,3 N 2,6

$b_4$) Ddz-Cys(Trt)-OTcp

Zu einer Lösung von 5,9 g (ca. 10 mmol) Ddz-Cys(Trt)-OH und 2,2 g (11 mmol) 2,4,5-Trichlorphenol in 50 ml absolutem Tetrahydrofuran gibt man bei 0°C 2,3 g DCC, gelöst in 15 ml Tetrahydrofuran, und rührt 2 Stunden bei 0°C und läßt über Nacht bei Raumtemperatur stehen. Der DC-Harnstoff wird abgesaugt und die Lösung eingeengt. Der Rückstand wird in Methylenchlorid gelöst und über 100 g

Kieselgel 60 filtriert. Man eluiert mit Methylenchlorid. Ausbeute 6,2 g amorphes Produkt (81%).
$[\alpha]_D^{22} = +10,3°$ (c = 1, in Methanol)

$C_{40}H_{36}Cl_3NO_6S$ (765,17)
Ber. C 62,79 H 4,74 N 1,83 S 4,19
Gef. C 61,0 H 4,7 N 1,7 S 4,2

Laut CHN-Analyse dürfte die Substanz 97%ig sein.

$b_5$) Ddz-Cys(Trt)-Thr(Bu$^t$)-Ser(Bu$^t$)-OH

Zu einer Lösung von 31,8 g (0,1 mol) H-Thr(Bu$^t$)-Ser(Bu$^t$)-OH (oder entsprechend mehr je nach Wasser oder Säuregehalt) und 13,5 g HOBt (0,1 mol) in 100 ml Dimethylformamid gibt man bei 0°C 12,8 ml (0,1 mol) N-Äthylmorpholin und 76,5 g (0,1 mol) Ddz-Cys(Trt)-OTcp. Anschließend wird ca. 6 Std. bei Raumtemperatur gerührt und die Lösung im Hochvakuum eingeengt. Der Rückstand wird zwischen Eis-Wasser und Essigester verteilt. Die Essigesterphase wird mit $NaHCO_3$-Lösung ausgeschüttelt, wobei Ddz-Cys(Trt)-Thr(Bu$^t$)-Ser(Bu$^t$)-OH als Natriumsalz in der Essigesterphase bleibt. Danach wird bei 0°C mit 100 ml 1n Zitronensäure und 100 ml Citratpuffer (pH 3) ausgeschüttelt und mit Wasser neutral gewaschen. Die Essigesterphase wird über $Na_2SO_4$ getrocknet, eingeengt und der Rückstand im Hochvakuum getrocknet. Zur weiteren Reinigung wird die Substanz aus Äther/Petroläther umgefällt, wobei die ätherische Lösung in den vorgelegten Petroläther langsam eingetropft wird. Ausbeute 44,5 g (50%). Schmp. 116—128° (Zers.).
$[\alpha]_D^{22} = +25,5°$ (c = 1, in Methanol),

$C_{49}H_{63}N_3O_{10}S$ (886,13)
Ber. C 66,42 H 7,17 N 4,74
Gef. C 66,1 H 7,4 N 4,7

$b_6$) H-Cys(Trt)-Thr(Bu$^t$)-Ser(Bu$^t$)-OH-trifluoracetat

8,8 g (10 mmol) Ddz-Cys(Trt)-Thr(Bu$^t$)-Ser(Bu$^t$)-OH werden bei Raumtemperatur in einer Mischung aus 8,75 ml (0,1 mol) Trifluoressigsäure, 1,75 ml Wasser und 165 ml Methylenchlorid (= ca. 175 ml 5%ige Trifluoressigsäure in Methylenchlorid mit 1% Wasser) gelöst. Man läßt 1,5 Std. bei Raumtemperatur stehen und stellt mit 8,85 ml (0,11 mol) Pyridin neutral. Die Lösung wird eingeengt und der Rückstand zwischen Essigester und Wasser verteilt. Die Essigesterphase wird noch zweimal mit Wasser gewaschen, über $Na_2SO_4$ getrocknet und eingeengt. Der Rückstand wird in wenig Äther gelöst und langsam in Petroläther eingetropft. Ausbeute 4,2 g (54%).
Schmp. 85—95° (Zers.).
$[\alpha]_D^{22} = +68,5°$ (c = 1, in Methanol).

$C_{37}H_{49}N_3O_6S \cdot CF_3COOH$ (777,9)
Ber. C 60,21 H 6,48 N 5,40 S 4,12
Gef. C 60,7 H 6,7 N 5,2 S 4,2

$b_7$) Ddz-Cys(Trt)-Cys(Trt)-Thr(Bu$^t$)-Ser(Bu$^t$)-OH

Zu einer Lösung von 5,3 g (6,8 mmol) H-Cys(Trt)-Thr(Bu$^t$)-Ser(Bu$^t$)-OH-trifluoracetat und 0,95 g (0,7 mmol) HOBt in 15 ml Dimethylformamid gibt man bei 0° 0,9 ml (0,7 mmol) N-Äthyl-morpholin und 5,35 g (7 mmol) Ddz-Cys(Trt)OTcp. Man rührt 14 Stunden bei Raumtemperatur und engt die Lösung ein, wenn das Tripeptid abreagiert ist. (Sollte noch unreagiertes Tripeptid nachweisbar sein, muß noch etwas Ddz-Cys(Trt)-OTcp zugesetzt werden). Den Rückstand nimmt man in Essigester auf. Die Essigesterphase wird nacheinander mit 10—20 ml gesättigter $NaHCO_3$-Lösung, mit 7 ml 1n Zitronensäure und 10 ml Zitratpuffer und mit NaCl-Lösung neutral gewaschen, über $Na_2SO_4$ getrocknet und eingeengt. Der Rückstand wird mit Petroläther verrieben.

Zur weiteren Reinigung wird aus Äther/Petroläther gefällt oder mit Diisopropyläther verrührt. Auch aus Methanol/Wasser kann umgefällt werden. Ausbeute 6 g (71%). Schmp. 118—130° (Zers.). $[\alpha]_D^{22} = +11,6°$ (c=1, in Methanol).

$C_{71}H_{82}N_4O_{11}S_2$ (1231,6)
Ber. C 69,23 H 6,71 N 4,55 S 5,21
Gef. C 67,2 H 6,3 N 4,1 S 5,3

Laut CHN-Analyse ist die Substanz nur etwa 97%ig.

Beispiel 8

a) H-Cys($SO_3H$)-Cys($SO_3H$)-Thr-Ser-Ile-Cys($SO_3H$)-Ser-Leu-Tyr-Gln-Leu-Glu-Asn-Tyr-Cys($SO_3H$)-Asn-OH

983 mg (0,34 mMol) H-Cys(Trt)-Cys(Trt)-Thr($Bu^t$)-Ser($Bu^t$)-Ile-Cys(Trt)-Ser-Leu-Tyr($Bu^t$)-Gln-Leu-Glu($OBu^t$-Asn-Tyr($Bu^t$-Cys(Trt)-Asn-$OBu^t$-trifluoracetat werden in einer Mischung aus 7,5 ml Äthylmercaptan und 7,3 ml Trifluoressigsäure gelöst. Man läßt 4 Stunden bei Raumtemperatur rühren und gießt die Reaktionsmischung in 50 ml Diäthyläther. Es entsteht ein Niederschlag, der abgesaugt wird. Der Nutschrückstand wird mit Äther gewaschen und getrocknet. Ausbeute 541 mg.

Da im NMR-Spektrum noch ein kleines Tritylsignal sichtbar war, wurden mit 420 mg der oben gewonnenen Substanz die Abspaltung noch einmal wiederholt. Ausbeute 506,7 mg. Zur besseren Charakterisierung wurde das SH-Peptid in das S-Tetrasulfonat überführt: 450 mg der obigen Substanz werden in 19,6 ml Tris-HCl-Puffer (0,1 m, pH 7,19) suspendiert. Nach Zugabe von 9,16 g Harnstoff läßt man 10 Minuten rühren und gibt bei Raumtemperatur 621 mg Natriumsulfit und 621 mg Natriumtetrathionat zu. Es entsteht eine klare Lösung. Nach 3 Stunden Reaktionszeit bei Raumtemperatur stellt man über Nacht in den Kühlraum (4° C). Anderntags wird der Ansatz gegen 5mal 5 Liter Wasser dialysiert und gefriergetrocknet. Ausbeute 438,9 mg (ca. 51%, bezogen auf geschützten Peptid und Proteingehalt). $[\alpha]_D^{26} = -51°$ (c=1, in Wasser).

Das NMR-Spektrum zeigt kein Signal für Tritylprotonen. In der sauren Elektrophorese läuft das Tetrasulfonat einheitlich und etwas schneller als das entsprechende Tetrasulfonat der Human-Insulin-A-Kette.

$C_{76}H_{117}N_{19}O_{39}S_8$ (2177,5)

Aminosäureanalyse:

| | | | | | | | | |
|---|---|---|---|---|---|---|---|---|
| Ber. | Asp 2,0 | Thr 1,0 | Ser 2,0 | Glu 2,0 | Cys 4,0 | Ile*) 1,0 | Leu 2,0 | Tyr 2,0 |
| Gef. | Asp 2,00 | Thr 0,96 | Ser 1,76 | Glu 1,88 | Cys 2,73 | Ile*) 1,17 | Leu 2,27 | Tyr 1,98 |

*) 26,5% davon sind D-allo-Ile.

Laut Aminosäureanalyse hat das Peptid ca. 69% Proteingehalt.

b) Herstellung der Ausgangssubstanz

H-Cys(Trt)-Cys(Trt)-Thr($Bu^t$)-Ser($Bu^t$)-Ile-Cys(Trt)-Ser-Leu-Tyr($Bu^t$)-Gln-Leu-Glu($OBu^t$)-Asn-Tyr($Bu^t$)-Cys(Trt)-Asn-$OBu^t$-trifluoracetat

$b_1$) Ddz-Cys(Trt)-Cys(Trt)-Thr($Bu^t$)-Ser($Bu^t$)-Ile-Cys(Trt)-Ser-Leu-Tyr($Bu^t$)-Gln-Leu-Glu($OBu^t$)-Asn-Tyr($Bu^t$)-Cys(Trt)-Asn-$OBu^t$

Zu einer Lösung von 16 g (7 mMol) H-Ile-Cys(Trt)-Ser-Leu-Tyr($Bu^t$)Gln-Leu-Glu($OBu^t$)-Asn-Cys-Tyr($Bu^t$)-Cys(Trt)-Asn-$OBu^t$-trifluoracetat, 9,5 g (7,7 mMol) Ddz-Cys(Trt)-Cys(Trt)-Thr($Bu^t$)-Ser($Bu^t$)-OH, 1,04 g (7,7 mMol) HOBt und 0,9 ml (7 mMol) N-Äthylmorpholin in 70 ml einer Dimethylformamid/Dimethylacetamid-Mischung (1 : 1) gibt man bei 0° C 1,7 g (8,25 mMol) DCC. Man läßt 2 Stunden bei 0° C und anschließend bei Raumtemperatur rühren. Man läßt über Nacht stehen und rührt anderntags noch einmal 5 Stunden bei Raumtemperatur. Der ausgefallene DC-Harnstoff wird abgesaugt. Das Filtrat wird auf die Hälfte am Hochvakuum eingeengt und anschließend unter Rühren in Eiswasser eingetropft. Der Niederschlag wird abgesaugt und nacheinander mit $NaHCO_3$-Lösung, Zitratpuffer (pH 3), $NaHCO_3$-Lösung und Wasser gewaschen und über $P_2O_5$ getrocknet. Zur weiteren Reinigung wird mit 100 ml Essigester unter Erwärmen verrührt. Nach Zugabe von 200 ml Äther wird der Niederschlag noch warm abgesaugt und getrocknet. Anschließend wird die Substanz mit 220 ml 50proz. wäßrigem Methanol und zum Schluß mit 200 ml Methanol erhitzt und jeweils heiß abgesaugt. Ausbeute 19,42 g (81%), Schmp. 250–260° (Zers.). $[\alpha]_D^{22} = -14,2°$ (c=1, in Dimethylformamid).

$C_{188}H_{235}N_{19}O_{31}S_4 \cdot 2\ H_2O$ (3421,4)

| | | | | |
|---|---|---|---|---|
| Ber. | C 66,00 | H 7,04 | N 7,78 | S 3,75 |
| Gef. | C 65,4 | H 7,1 | N 8,0 | S 3,8 |

Aminosäureanalyse (nach Oxydation von Cystein zur Cysteinsäure):

| | | | | | | | | |
|---|---|---|---|---|---|---|---|---|
| Ber. | Asp 2,0 | Thr 1,0 | Ser 2,0 | Glu 2,0 | Cys 4,0 | Ile 1,0 | Leu 2,0 | Tyr 2,0 |
| Gef. | Asp 1,99 | Thr 0,89 | Ser 1,69 | Glu 1,92 | Cys 3,97 | Ile 0,87 | Leu 2,07 | Tyr — |

Tyrosin fehlt, da es bei der Oxydation zerstört wird.

b2) H-Cys(Trt)-Cys(Trt)-Thr(Bu^t)-Ser(Bu^t)-Ile-Cys(Trt)-Ser-Leu-Tyr(Bu^t)-Gln-Leu-Glu(OBu^t)-Asn-Tyr(Bu^t)-Cys-(Trt)-Asn-OBu^t-trifluoracetat

10,26 g (3 mMol) Ddz-Cys(Trt)-Cys(Trt)-Thr(Bu^t)-Ser(Bu^t)-Ile-Cys(Trt)-Ser-Leu-Tyr(Bu^t)-Gln-Leu-Glu(OBu^t)-Asn-Tyr(Bu^t)-Cys(Trt)-Asn-OBu^t werden in einer Mischung aus 5,25 ml (60 mMol) Trifluoressigsäure, 1,05 ml Wasser und 99 ml Methylenchlorid (= ca. 105 ml einer 5proz. Trifluoressigsäure-Methylenchlorid-Lösung mit 1% Wasser) und 10,5 ml Anisol unter Rühren gelöst.

Man läßt 4 Stunden bei Raumtemperatur rühren. Danach versetzt man mit 10,5 ml Pyridin und engt ein. Im Hochvakuum wird nachdestilliert. Der ölige Rückstand wird mit Äther verrieben und der Niederschlag abgesaugt und getrocknet. Anschließend wird die Substanz mit Wasser gewaschen und über $P_2O_5$ getrocknet. Zur weiteren Reinigung wird die Substanz dreimal mit je 100 ml Essigester aufgekocht und jeweils heiß abgesaugt. Ausbeute 7,96 g (81%), Schmp. 240–245° (Zers.). $[\alpha]_D^{23} = -10{,}0°$ (c = 1, in Dimethylformamid).

$C_{176}H_{221}N_{19}O_{27}S_4 \cdot CF_3COOH$ (3277,15)

Aminosäureanalyse:

| | | | | | | | | |
|---|---|---|---|---|---|---|---|---|
| Ber. | Asp 2,0 | Thr 1,0 | Ser 2,0 | Glu 2,0 | Cys 4,0 | Ile 1,0 | Leu 2,0 | Tyr 2,0 |
| Gef. | Asp 1,96 | Thr 0,80 | Ser 1,49 | Glu 2,03 | Cys 3,21 | Ile 1,10 | Leu 2,04 | Tyr 1,78 |

Beispiel 9

a) H-Gly-Ile-Val-Glu-Gln-Cys($SO_3H$)-Cys($SO_3H$)-Thr-Ser-Ile-Cys($SO_3H$)-Ser-Leu-Tyr-Gln-Leu-Glu-Asn-Tyr-Cys($SO_3H$)-Asn-OH (= Humaninsulin-A-Kette-tetrasulfonat)

772 mg (0,2 mMol) H-Gly-Ile-Val-Glu(OBu^t)-Gln-Cys(Trt)-Cys(Trt)-Thr(Bu^t)-Ser(Bu^t)-Ile-Cys(Trt)-Ser-Leu-Tyr-(Bu^t)-Gln-Leu-Glu(OBu^t)-Asn-Tyr(Bu^t)-Cys(Trt)-Asn-Obu^t · $CF_3COOH$ werden in einer Mischung aus 5 ml Äthylmercaptan und 5 ml Trifluoressigsäure suspendiert. Man rührt 4 Stunden bei Raumtemperatur und schüttet in 50 ml Äther. Der ausgefallene Niederschlag wurde abgesaugt und mit Äther gewaschen. Ausbeute 542 mg. Diese Operation wurde mit der 542-mg-Substanz wiederholt. Ausbeute 385,9 mg. Bei einem Gehalt von 80% Protein entspricht dies einer Ausbeute von ca. 65%. $C_{99}H_{155}N_{25}O_{35}S_4$ (2383,8).

Zur Charakterisierung wurden aus 360 mg Substanz analog Beispiel 8a das Tetrasulfonat hergestellt. Ausbeute 370,8 mg (ca. 60%, bezogen auf geschützte Insulin-A-Kette). $[\alpha]_D^{21} = -72{,}9°$ (c = 1, in Wasser).

$C_{99}H_{155}N_{25}O_{47}S_8$ (2704)

Aminsäureanalyse:

| | | | | | | | | | | |
|---|---|---|---|---|---|---|---|---|---|---|
| Ber. | Asp 2,0 | Thr 1,0 | Ser 2,0 | Glu 4,0 | Gly 1,0 | Cys 4,0 | Val 1,0 | Ile 2,0 | Leu 2,0 | Tyr 2,0 |
| Gef. | Asp 1,84 | Thr 0,90 | Ser 1,62 | Glu 4,04 | Gly 0,94 | Cys 3,48 | Val 0,89 | Ile 1,81*) | Leu 2,20 | Tyr 2,2 |

*) Summe aus 0,44-D-allo-Ile und 1,37 Ile

b) Herstellung der Ausgangssubstanz H-Gly-Ile-Val-Glu-(OBu^t)-Gln-Cys(Trt)-Cys(Trt)-Thr(Bu^t)-Ser(Bu^t)-Ile-Cys-(Trt)-Ser-Leu-Tyr(Bu^t)-Gln-Leu-Glu(OBu^t)-Asn-Tyr(Bu^t)-Cys-(Trt)-Asn-OBu^t · $CF_3COOH$

b1) Ddz-Val-OTcp

Zu einer Lösung von 113,3 g (0,334 mMol) Ddz-Val-OH und 65,9 g (0,334 mMol) 2,4,5-Trichlorphenol in 300 ml Essigester gibt man bei 0° C eine Lösung von 72,1 g (0,35 mMol) DCC in 100 ml Essigester. Man läßt 1 Stunde bei 0° und 3 Stunden bei Raumtemperatur rühren. Der DC-Harnstoff wird abgesaugt und das Filtrat eingeengt. Der Rückstand wird mit Petroläther kristallisiert. Ausbeute 111,45 g (64%), Schmp. 73–76°, $[\alpha]_D^{25} = -28{,}7°$ (c = 1, in Methanol).

$C_{23}H_{26}NO_6Cl_3$ (518,84)

| | | | |
|---|---|---|---|
| Ber. | C 53,24 | H 5,05 | N 2,70 |
| Gef. | C 53,5 | H 5,2 | N 2,9 |

b2) Ddz-Val-Glu(OBu^t)-OH

Zu einer Lösung von 30,5 g (150 mMol) H-Glu(OBu^t)-OH und 20,25 g (150 mMol) HOBt in 200 ml Dimethylformamid gibt man 19,2 ml (150 mMol) N-Äthylmorpholin und 77,8 g Ddz-Val-OTcp. Man läßt 4 Stunden bei Raumtemperatur rühren. Ist im DC kein H-Glu(OBu^t)-OH mehr nachweisbar, wird im Hochvakuum eingeengt. (Andernfalls bringt man durch Zusatz von wenig Ddz-Val-OTcp die Reaktion zu Ende.) Das resultierende Öl wird zwischen 300 ml Essigester und 300 ml Wasser verteilt. Die

Essigesterphase wird zweimal mit je 150 ml Citratpuffer (pH 3) und einmal mit 150 ml Wasser ausgeschüttelt, über $Na_2SO_4$ getrocknet und eingeengt. Ausbeute 138,7 g (78,7 g = 100%). Das resultierende Ol wird ohne weitere Reinigung und Charakterisierung als 150 mMol weiter umgesetzt. $C_{26}H_{40}N_2O_9$ (524,6).

b3) Ddz-Val-Glu(OBu$^t$)-Gln-ONb

Zu einer Lösung von rohem, verunreinigtem 138,7 g (=150 mMol) Ddz-Val-Glu(OBu$^t$)-OH, 47,65 g (150 mMol) H-Gln-ONb · HCl und 20,25 g (150 mMol) HOBt in 450 ml Dimethylformamid gibt man bei 0° C 19,2 ml (150 mMol) N-Äthylmorpholin und 33 g (160 mMol) DCC. Man läßt 1 Stunde bei 0° C und 4 Stunden bei Raumtemperatur rühren. Man läßt über Nacht bei Raumtemperatur stehen, saugt den Niederschlag ab und engt das Filtrat im Hochvakuum ein. Das resultierende Öl wird zwischen 300 ml Essigester und 300 ml Wasser verteilt. Die Essigesterphase wird nacheinander mit gesättigter $NaHCO_3$-Lösung, Citratpuffer (pH 3), gesättigter $NaHCO_3$-Lösung und Wasser ausgeschüttelt, über $Na_2SO_4$ getrocknet und eingeengt. Das Öl kristallisiert mit Äther. Mit Äther wird gewaschen. Ausbeute 110,95 g (94%), Schmp. 135 – 138°, 138 – 142°.
$[\alpha]_D^{22} = -49,4°$ (c = 1, in Methanol).

$C_{38}H_{53}N_5O_{13}$ (787,9)
Ber. C 57,93 H 6,78 N 8,89
Gef. C 57,8 H 7,0 N 9,2

b4) H-Val-Glu-(OBu$^t$)-Gln-ONb-trifluoracetat

39,4 g (50 mMol) Ddz-Val-Glu(OBu$^t$)-Gln-ONb werden in einer Mischung aus 875 ml einer 5%igen Trifluoressigsäure-Methylenchlorid-Mischung (mit 1% Wasser) (=500 mMol Trifluoressigsäure) und 87,5 ml Anisol gelöst. Man läßt 30 Minuten bei Raumtemperatur stehen, neutralisiert mit 50 ml Pyridin und engt ein. Im Hochvakuum wird nachgetrocknet. Der Rückstand wird zwischen Essigester und Wasser verteilt. Die Essigesterphase wird noch zweimal mit wenig Wasser ausgeschüttelt, über $Na_2SO_4$ getrocknet und eingeengt. Der Rückstand wird mit Äther verrieben und im Hochvakuum getrocknet. Ausbeute 23,6 g amorphe Substanz (69%). $[\alpha]_D^{24} = -5,3°$ (c = 1, in Methanol).

$C_{26}H_{39}N_5O_9 \cdot CF_3COOH$ (679,67)
Ber. C 49,48 H 5,93 N 10,30
Gef. C 50,0 H 5,8 N 10,1

b5) Ddz-Gly-Ile-OMe

Zu einer Lösung von 14,54 g (80 mMol) H-Ile-OMe · HCl und 10,8 g (80 mMol) HOBt in 50 ml Dimethylformamid gibt man bei –3° C 10,3 ml (80 mMol) N-Äthylmorpholin und bei 0° C 40 g (84 mMol) Ddz-Gly-OTcp. Man läßt 3 Stunden bei Raumtemperatur rühren, und läßt über Nacht stehen. (Im DC war anderntags noch etwas H-Ile-OMe feststellbar. Es wurden daher nochmals 2,86 g [6 mMol] Ddz-Gly-OTcp zugegeben und gerührt. Nach 5 Stunden war kein H-Ile-OMe feststellbar.)

Die im DC von H-Ile-OMe freie Lösung wird im Hochvakuum eingeengt und das resultierende Öl zwischen Essigester und Wasser verteilt. Die Essigesterlösung wird nacheinander mit gesättigter $NaHCO_3$-Lösung, Citratpuffer (pH 3), $NaHCO_3$-Lösung und Wasser gewaschen und über $Na_2SO_4$ getrocknet. Die Essigesterlösung wird über 400 g basisches $Al_2O_3$ filtriert, das Eluat eingeengt und am Hochvakuum getrocknet. Ausbeute 30,6 g zähes Öl (90%). $[\alpha]_D^{25} = -6,8°$ (c = 1, in Methanol).

$C_{21}H_{32}N_2O_7$ (424,5)
Ber. C 59,42 H 7,60 N 6,60
Gef. C 59,1 H 7,7 N 6,3

b6) Ddz-Gly-Ile-OH

10 g (23,56 mMol) Ddz-Gly-Ile-OMe werden in einer Mischung aus 24 ml Dioxan und 8 ml Wasser gelöst und gegen Thymolphthalein als Indikator mit 1 n-NaOH titriert. Verbrauch je nach Essigestergehalt mehr als 23 ml. Nach Neutralisation mit Zitronensäure wird das Dioxan abdestilliert. Der Rückstand wird in Wasser aufgenommen und bei 0° C mit 2 n-Zitronensäure auf pH 3 gestellt. Das ausfallende Öl wird mit Essigester extrahiert. Die wäßrige Phase wird, wenn nötig nochmals auf pH 3 angesäuert und mit Essigester extrahiert. Die vereinigten Essigesterextrakte werden mit Wasser

einmal ausgeschüttelt, über $Na_2SO_4$ getrocknet und eingeengt. Im Hochvakuum wird getrocknet. Die Substanz wird amorph. Ausbeute 8,9 g (92%). Schmp. 80–90°. $[\alpha]_D^{23} = +4,2°$ (c=1, in Methanol).

$C_{20}H_{30}N_2O_7$ (410,48)
Ber. C 58,52 H 7,37 N 6,83
Gef. C 58,8 H 7,5 N 6,5

b₇) Ddz-Gly-Ile-Val-Glu(OBu[t])-Gln-ONb

Zu einer Lösung von 15,8 g (38,5 mMol) Ddz-Gly-Ile-OH, 23,8 g (35 mMol) H-Val-Glu(OBu[t])-Gln-ONb-trifluoracetat und 5,2 g (38,5 mMol) HOBt in 60 ml Dimethylformamid gibt man bei 0°C 4,48 ml (35 mMol) N-Äthylmorpholin und 8,47 g (41 mMol) DCC. Man läßt eine Stunde bei 0°C, mehrere Stunden bei Raumtemperatur rühren und über Nacht bei Raumtemperatur stehen. Es bildet sich ein Gelee, das in gekühlte $NaHCO_3$-Lösung eingerührt wird. Das Produkt wird abgesaugt und nacheinander mit Citratpuffer (pH 3), $NaHCO_3$-Lösung und Wasser verrieben und jeweils abgesaugt.
Über $P_2O_5$ wird getrocknet. Ausbeute 40,3 g.
Zur weiteren Reinigung wird aus 500 ml Äthanol umkristallisiert. Ausbeute 21,45 g (64%), Schmp. 205–207°, $[\alpha]_D^{25} = -12,3°$ (c=1, in Dimethylformamid).
Ein kleinerer Ansatz mit geringerer Ausbeute hatte folgende Daten: Schmp. 212–214°, $[\alpha]_D^{25} = -16,1°$ (c=1, in Dimethylformamid).

$C_{46}H_{67}N_7O_{15}$ (958,1)
Ber. C 57,57 H 7,05 N 10,23
Gef. C 57,6 H 7,2 N 10,0

b₈) Ddz-Gly-Ile-Val-Glu(OBu[t])-Gln-OH

19,16 g (20 mMol) Ddz-Gly-Ile-Val-Glu-(OBu[t])-Gln-ONB werden in einer Mischung von 100 ml Dimethylformamid und 100 ml Methanol gelöst. Nach Zugabe von $Pd/BaSO_4$ wird katalytisch hydriert. Nach 6 Stunden war alles hydriert. Die Suspension wird auf ca. 70°C erhitzt und in der Wärme vom Katalysator abgesaugt. Mit warmem Dimethylformamid wird nachgewaschen. Das Filtrat wird eingeengt und der Rückstand mit 100 ml Methanol verrieben. Man läßt über Nacht im Kühlraum stehen, saugt ab und wäscht mit kaltem Methanol nach. Trocknen im Vakuum über $P_2O_5$. Ausbeute 14 g (85%), Schmp. 238–241° (Zers.). $[\alpha]_D^{29} = -13,3°$ (c=1, in Dimethylformamid).

$C_{39}H_{62}N_6O_{13}$ (822,97)
Ber. C 56,92 H 7,59 N 10,21
Gef. C 56,0 H 7,6 N 10,8

b₉) Ddz-Gly-Ile-Val-Glu(OBu[t])-Gln-Cys(Trt)-Cys(Trt)-Thr(Bu[t])-Ser(Bu[t])-Ile-Cys(Trt)-Ser-Leu-Tyr(Bu[t])-Gln-Leu-Glu(OBu[t])-Asn-Tyr(Bu[t])-Cys(Trt)-Asn-OBu[t]

Zu einer Lösung von 3,28 g (1 mMol) H-Cys(Trt)-Cys(Trt)-Thr(Bu[t])-Ser(Bu[t])-Ile-Cys(Trt)-Ser-Leu-Tyr(Bu[t])-Gln-Leu-Glu(OBu[t])-Asn-Tyr(Bu[t])-Cys(Trt)-Ans-OBu[t]-trifluoracetat, 1 g (ca. 1,2 mMol) Ddz-Gly-Ile-Val-Glu(OBu[t])-Gln-OH und 162 mg HOBt (1,2 mMol) in 20 ml einer Mischung aus Dimethylformamid und Dimethylacetamid (1 : 1) gibt man 0,13 ml N-Äthylmorpholin und bei 0°C 310 mg (1,5 mMol) DCC. Man läßt 1 Stunde bei 0°C und 6 Stunden bei Raumtemperatur rühren und läßt über Nacht stehen. Es entsteht ein dickes Gelee, das mit Eiswasser versetzt wird. Der sich bildende Niederschlag wird abgesaugt und nacheinander mit $NaHCO_3$-Lösung, Citratpuffer (pH 3), $NaHCO_3$-Lösung und Wasser verrieben und jeweils abgesaugt. Mit Wasser wird gewaschen und über $P_2O_5$ getrocknet. Zur weiteren Reinigung wird 2mal mit je 25 ml Essigester ausgekocht und heiß abgesaugt. Ausbeute 2,87 g (72%). Schmp. 260–278° (Zers.). $[\alpha]_D^{24} = -24,5°$ (c=1, Dimethylformamid).

$C_{215}H_{281}N_{25}O_{39}S_4$ (3968,07)
Aminosäureanalyse:

| | | | | | | | | | | |
|---|---|---|---|---|---|---|---|---|---|---|
| Ber. | Asp 2,0 | Thr 1,0 | Ser 2,0 | Glu 4,0 | Gly 1,0 | Cys 4,0 | Val 1,0 | Ile 2,0 | Leu 2,0 | Tyr 2,0 |
| Gef. | Asp 2,06 | Thr 0,88 | Ser 2,04 | Glu 3,97 | Gly 1,01 | Cys 2,59 | Val 0,60 | Ile 1,45 | Leu 1,93 | Tyr 1,93 |

$b_{10}$) H-Gly-Ile-Val-Glu(OBu$^t$)-Gln-Cys(Trt)-Cys(Trt)-Thr(Bu$^t$)-Ser(Bu$^t$)Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$-trifluoracetat

2,78 g (0,7 mMol) Ddz-Gly-Ile-Val-Glu(OBu$^t$)-Gln-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$ werden mit 2,5 ml Anisol in 25 ml einer 5proz. Trifluoressigsäure-Methylenchlorid-Lösung mit 1% Wasser (= ca. 14 mMol Trifluoressigsäure) 4 Stunden bei Raumtemperatur gerührt. Danach gibt man 1,4 ml Pyridin zu und engt zuerst am Wasserstrahlpumpenvakuum und zum Schluß an Hochvakuum ein. Der Rückstand wird mit Äther verrieben, abgesaugt und mit Äther gewaschen und getrocknet. Die Substanz wird anschließend mit Wasser verrieben, abgesaugt, mit Wasser gewaschen und über $P_2O_5$ getrocknet. Zur weiteren Reinigung wird zweimal mit je 50 ml Essigester aufgekocht und heiß abgesaugt. Ausbeute 1,85 g (68,5%), Schmp. 275–280° (Zers.), $[\alpha]_D^{23} = -24,0°$ (c=1, in Dimethylformamid).

$C_{203}H_{267}N_{25}O_{35}S_4 \cdot CF_3COOH$ (3859,8)
Aminosäureanalyse:

| | | | | | | | | | | |
|---|---|---|---|---|---|---|---|---|---|---|
| Ber. | Asp 2,0 | Thr 1,0 | Ser 2,0 | Glu 4,0 | Gly 1,0 | Cys 4,0 | Val 1,0 | Ile 2,0 | Leu 2,0 | Tyr 2,0 |
| Gef. | Asp 2,05 | Thr 1,08 | Ser 1,80 | Glu 4,00 | Gly 0,97 | Cys 2,40 | Val 0,71 | Ile 1,51 | Leu 2,06 | Tyr 1,88 |

**Patentanspruch**

Verfahren zur Herstellung von cysteinhaltigen Peptiden durch Abspaltung der S-Tritylgruppen mittels Trifluoressigsäure, dadurch gekennzeichnet, daß man S-Tritylverbindungen dieser Peptide mit einer Mischung aus einem Mercaptan und Trifluoressigsäure bei einer Trifluoressigsäurekonzentration von mindestens 30 Volumprozent behandelt.

**Claim**

A process for the preparation of cysteine-containing peptides by detachment of the S-trityl groups by means of trifluoroacetic acid, wherein S-trityl compounds of these peptides are treated with a mixture of a mercaptan and trifluoroacetic acid at a concentration of trifluoroacetic acid of at least 30 per cent by volume.

**Revendication**

Procédé de préparation de peptides contenant de la cystéine, par séparation des groupes S-trityle au moyen d'acide trifluoroacétique, caractérisé en ce qu'on traite les dérivés S-trityle de ces peptides avec un mélange d'un mercaptan et d'acide trifluoroacétique, avec une concentration en acide trifluoroacétique d'au moins 30% en volume.